# EUROPÄISCHE PATENTSCHRIFT

(11) **EP 0 421 457 B1**
(45) Veröffentlichungstag und Bekanntmachung des Hinweises auf die Patenterteilung: **11.01.1995**
(21) Anmeldenummer: 90119130.4
(22) Anmeldetag: 05.10.1990
(51) Int. Cl.: A61B 17/32, A61B 17/22

(54) **Vorrichtung zur Entfernung von Ablagerungen in Gefässen**
Device for removal of deposits in vessels
Dispositif pour l'enlèvement des dépôts dans les vaisseaux

(30) Priorität: 06.10.1989 DE 8911911 U; 06.10.1989 DE 8911909 U; 06.10.1989 DE 8911912 U
(43) Veröffentlichungstag der Anmeldung: 10.04.1991
(73) Patentinhaber: ANGIOMED AG, 76227 Karlsruhe (DE)
(72) Erfinder: Schnepp-Pesch, Wolfram, W-7505 Ettlingen (DE); Lindenberg, Josef, W-7500 Karlsruhe (DE)
(74) Vertreter: Dipl.-Ing. Heiner Lichti Dipl.-Phys. Dr.rer.nat. Jost Lempert Dipl.-Ing. Hartmut Lasch

(56) Entgegenhaltungen:
- EP-A- 0 379 783
- WO-A-80/02499
- DE-U- 8 900 971
- GB-A- 2 093 353
- GB-B- 790 267
- US-A- 4 030 503
- US-A- 4 857 046

## Beschreibung

Die Erfindung betrifft eine Vorrichtung zur Entfernung von Ablagerungen, wie Plaques in Gefäßen, arteriosklerotisch umgewandelte Wandpartien oder dergleichen, mit einer sich am vorderen Ende eines drehbaren Drahtes erstreckenden Arbeitswendel, die an ihrem distalen Ende mit einer Kugel versehen.

Unter vorderem Ende ist das in den Körper zuerst eingeführte Ende des jeweiligen Teils zu verstehen (proximal zum Körperinneren des Patienten, distal zum behandelnden Arzt).

Eine gattungsgemäße Vorrichtung ist aus der DE-GM 89 00 971 bekannt. Die bekannte Vorrichtung arbeitet weitgehend zufriedenstellend, doch sind Verbesserungen insbesondere zum Einsatz einer solchen Vorrichtung für die Atherektomie wünschenswert.

Während durch die Vorrichtung nach der DE-GM 89 00 971 die Entfernung der Plaques, insbesondere von festen und rigiden Plaques, durch das dort bewirkte Schlagen der Wendel im Bereich der Ablagerungen in zufriedenstellender Weise erfolgt, ist dies bei eher zähen arteriosklerotisch umgewandelten Wandbereichen nicht in wünschenswertem Maß der Fall.

Aus der US-A-4 857 046 ist ein angetriebener Katheter mit einer Kugel bekannt, die auf ihrer Außenseite sich über die gesamte Höhe der Kugel erstreckende Rippen und zwischen diesen ausgebildete Nuten aufweist, mit der Ablagerungen in Blutgefäßen abgeschliffen werden können. Es besteht die Gefahr, daß die abgetragenen Partikel zu klein sind, so daß sie bis in feinste Blutgefäße dringen und dort zu Schädigungen führen können. Die Vorrichtung kann im übrigen auch nur harte Plaques entfernen, nicht aber zähere arteriosklerotisch umgewandelte Wandpartien.

Der Erfindung liegt daher die Aufgabe zugrunde, eine Vorrichtung der eingangs genannten Art dahingehend weiterzuentwickeln, daß sie ohne Verursachung neuer Gefahren auch bei zäheren arteriosklerotisch umgewandelten Wandpartien eine kontrollierte, über den Umfang symmetrische Entfernung von belastendem, den Strömungswiderstand im Gefäß erhöhendem Material ermöglicht.

Erfindungsgemäß wird die genannte Aufgabe bei einer Vorrichtung der eingangs genannten Art durch die kennzeichnenden Merkmale des Anspruchs 1 gelöst.

Durch die scharfkantige Ausbildung des Außenumfangs der Kugel können die arteriosklerotisch umgewandelten Wandbereiche von Gefäßen, insbesondere Blutgefäßen, kontrolliert schneidend, zuverlässig und schnell entfernt werden, was durch das bei der bekannten Vorrichtung im wesentlichen gegebene einfache Schlagen der Arbeitswendel bzw. dem entsprechend ausgebildeten distalen Ende bei weniger rigiden und eher zähen Wandbereichen, wie dies bei arteriosklerotisch umgewandelten Bereichen der Fall ist, schlechter möglich war und insbesondere eine längere Zeit benötigte. Darüber hinaus kann durch die erfindungsgemäße Ausgestaltung eine kontrollierte symmetrische Erweiterung des Gefäßes erzielt werden. Dadurch, daß die Schneidkanten lediglich über einen Teil der Höhe der Kugel vorgesehen sind, wird die Gefahr ausgeschlossen, daß durch Schneidbereiche an den axialen Enden der Kugel unerwünschte Schädigungen auftreten.

Zu der Ausgestaltung des Antriebs einer entsprechenden erfindungsgemäßen Vorrichtung, der Durchführung der Antriebswelle durch einen Katheter oder dergleichen wird ausdrücklich auf die genannte DE-GM 89 00 971 verwiesen, deren Offenbarungsgehalt insofern ausdrücklich zum Offenbarungsgehalt der vorliegenden Patentschrift gemacht wird. Durch die erfindungsgemäße Ausgestaltung der Vorrichtung aus dem Gefäß entferntes Material kann in an sich bekannter Weise durch Aspiration über den genannten Katheter nach außen entfernt werden.

Eine bevorzugte Ausgestaltung sieht vor, daß die scharfen Kanten durch die in Drehrichtung der Kugel gesehene rückwärtige Übergangskante zwischen Außenmantel der Kugel und der Begrenzungswand eines unter einem spitzen Winkel zur Tangente an die Außenwandung in der Kugel gebildeten Schlitzes gebildet ist. Durch den durch die Schneidkante gebildeten spitzen Winkel zwischen dem Außenumfang der Kugel bzw. einer Tangente an diesem und der die Schneidkante mit dem Außenumfang bildenden Innenwandung des Schlitzes werden Gewebeablagerungen praktisch abgeschält, ohne daß die Gefahr tiefer Einschnitte in das lebende Gewebe des Gefäßes besteht. Dissektion oder antegrad hinterschnittene Taschen werden hierdurch vermieden.

Die Lösung der genannten Aufgabe durch die vorstehenden Maßnahmen wird unterstützt, wenn die Außenseite der Arbeitswendel ebenfalls eine scharfe Kante aufweist. Für die Wirkungen und Vorteile gilt das oben zu scharfkantigen Kugelausbildung Gesagte.

Bevorzugte Weiterbildungen der Außenkante der Arbeitswendel sehen vor, daß die scharfe Kante durch Zuschliff des Außenbereichs der Arbeitswendel gebildet ist oder daß die scharfe Kante durch Quetschung des Außenbereichs der Arbeitswendel gebildet ist. Die scharfen Kanten können dabei entweder bei einer Vorrichtung vorgesehen sein, bei der die Arbeitswendel durch einen schraubenförmig geführten Draht gebildet ist oder bei der die Arbeitswendel durch ein mehrfach um seine Längsachse verdrehtes Blatt gebildet ist.

Um bei kleinen Punktionsstellen einen großen radialen Arbeitsbereich zur Entferung von Ablagerungen in Gefäßen zu erzielen, sieht die Erfindung vor, daß die Kugel exzentrisch zur Achse des drehbaren Drahts angeordnet ist. Dies kann durch leichte Biegung der Arbeitswendel oder exzentrische Anordnung der Kugel erreicht werden.

Durch die exzentrische Führung der am vom Behandler abgewandten Ende der Arbeitswendel angebrachten Kugel wird eine kontrollierte Vergrößerung des Arbeitsdurchmessers von dem Durchmesser der Wendel selbst auf nahezu das Doppelte erreicht, ohne daß gegenüber dem für die Durchführung der Wendel erforderlichen Durchmesser des Führungskatheters und der diesem angepaßten Punktion eine Vergrößerung der Punktionsstelle erforderlich wäre. Durch die Führung wird darüber hinaus eine verbesserte kontrollierte Abrasion der Wandpartien erreicht. Das Risiko einer Dissektion oder antegraden Taschenbildung im Bereich der Gefäßwandungen wird reduziert bzw. vollständig eliminiert.

Die Erfindung sieht in Weiterbildung vor, daß die Kugel parallel zur Symmetrieachse der Arbeitswendel, aber exzentrisch zu dieser, eine Bohrung aufweist, durch die sich ein stationär gehaltener Führungsdraht erstreckt. Der Arbeitsdurchmesser wird über den Durchmesser der Arbeitswendel hinaus erweitert bis nahezu auf den doppelten Umfang derselben, wobei das gesamte Operationsmaterial, insbesondere also der Katheter, durch den die Arbeitswendel in das Gefäß eingeführt wird, kleinlumig bleiben kann. Bei unverändertem Katheter und entsprechend unveränderten kleinen Punktionsstellen kann durch die genannte Ausbildung eine großlumigere Öffnung im Gefäß erreicht werden, als dies bei einer gattungsgemäßen Vorrichtung bisher der Fall war. In dieser Weise kann der zu erweiternde Durchlaß über den Querschnitt des Operationsmaterials hinaus in gleicher Weise erweitert werden, wie dies bisher nur mit Ballonkathetern durch Aufblasen derselben in komplizierterer Weise möglich war.

Weitere Vorteile und Merkmale der Erfindung ergeben sich aus den Ansprüchen und aus der nachfolgenden Beschreibung, in der Ausführungsbeispiele der Erfindung unter Bezugnahme auf die Zeichnung im einzelnen erläutert sind. Dabei sind die einzeln bevorzugten Ausführungsformen der Erfindung lediglich zum besseren Verständnis in verschiedenen Figuren dargestellt und separat erläutert; sie können selbstverständlich weitgehend auch gemeinsam an einem Gegenstand verwirklicht sein. Es zeigt:
- Fig. 1: Eine erste Ausführungsform einer Vorrichtung mit einer an seiner Außenseite mit einer scharfen Kante versehenen Arbeitswendel;
- Fig. 2: einen Schnitt entsprechend II-II der Figur 1 zur Darstellung einer Ausbildung der Außenkante;
- Fig. 3: einen entsprechenden Schnitt mit einer anders ausgebildeten Außenkante;
- Fig. 4: eine Ausführungsform der Erfindung mit einem assymmetrisch eine distale Kugel durchstoßenden Führungsdraht;
- Fig. 5: eine mit erfindungsgemäßen Schneidkanten versehene distale Arbeitskugel in Aufsicht entsprechend dem Pfeil V der Figur 6;
- Fig. 6: eine Seitenansicht der Kugel der Figur 5 in Richtung des Pfeils VI der Figur 5;
- Fig. 7: eine Darstellung einer anderen Ausgestaltung der Arbeitswendel, die ebenfalls scharfe Außenkanten aufweisen kann;
- Fig. 8: einen Schnitt entsprechend VIII-VIII der Figur 7 durch die Arbeitswendel zur Darstellung der scharfen Außenkanten.

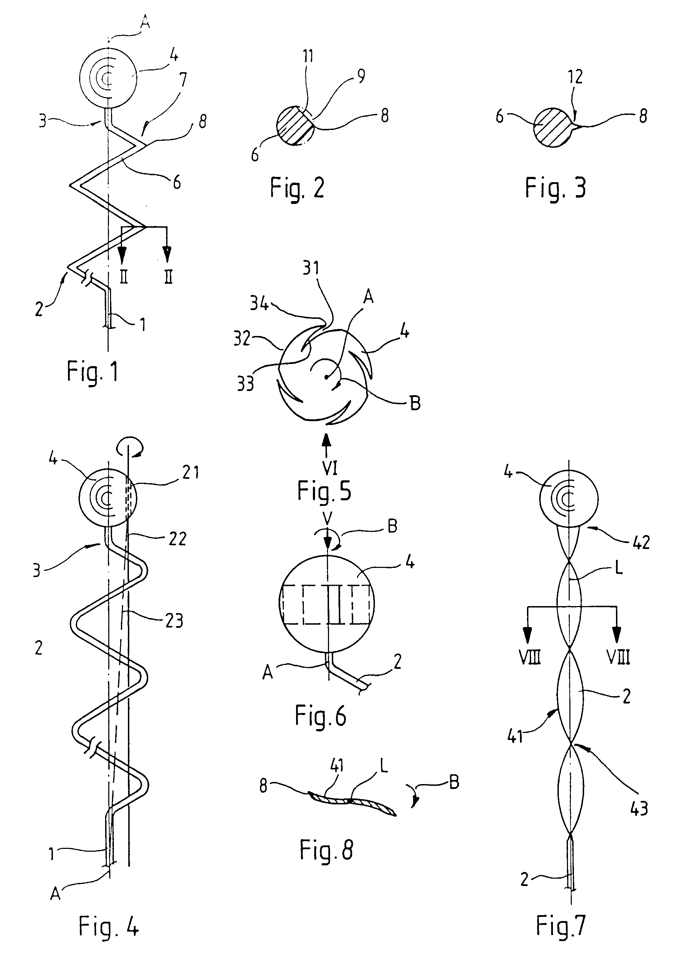

Die Figuren 5 und 6 zeigen erfindungsgemäße Ausbildungen des distalen Arbeitsendes einer Vorrichtung zum Entfernen von Ablagerungen, wie Plaques in Gefäßen, arteriosklerotisch umgewandelten Wandpartien derselben oder dergleichen, wie sie aus der DE-GM 89 00 971 bekannt ist. Eine erfindungsgemäße Vorrichtung weist eine Antriebswelle oder einen Führungsdraht 1 auf, an deren distalem Ende eine Arbeitswendel 2 ausgebildet ist, deren distales Ende 3 zur Vermeidung, daß sie Verletzungen bewirken kann, abgestumpft ist durch eine auf das vordere Ende des Drahtes aufgesetzte Kugel 4. Die Antriebswelle bzw. der Führungsdraht hat eine hinreichende Stärke zu einer kontrollierten Führung der sich exzentrisch um ihn drehenden Kugel. Zum Einsatz erstreckt sich die Antriebswelle 1 regelmäßig durch ein Führungsteil, wie einen Kunststoffkatheter, der zweilumig ausgebildet sein kann, wobei innerhalb des Führungskatheters gegebenenfalls auf der Welle 1 eine Schraubgewindewendel drehbar mit der Welle 1 zum Fördern entfernter Plaques-Teilchen vom distalen Ende 3 durch das Führungsteil (Katheter) zu dessen proximalem Ende und aus diesem heraus, beispielsweise über einen Abzweig, der an eine Absaugeinrichtung angeschlossen sein kann. Das proximale, also dem Behandler zugewandte Ende der Antriebswelle 1 ragt aus dem proximalem Ende des hohlen Führungsteils aus diesem heraus und ist vorzugsweise an der Abtriebswelle eines Motors befestigt, der die Antriebswelle 1 und damit die Arbeitswendel 2 antreibt. Diese Ausgestaltungen sind in den Figuren im einzelnen nicht dargestellt, da sie in der genannten Gebrauchsmusterschrift 89 00 971 eingehend erläutert sind, worauf ausdrücklich verwiesen wird und sie daher dem Fachmann bekannt sind.

In der Ausgestaltung der Figur 1 besteht die Arbeitswendel 2 aus einem wendelförmig über mehrere Windungen gebogenen Draht 6, der wie den Figuren 2,3 zu entnehmen ist, grundsätzlich ein Runddraht sein kann. Der Draht 6 und damit die Arbeitswendel 2 ist an ihrer Außenseite 7 mit einer scharfen Arbeitskante 8 versehen. Diese Arbeitskante 8 kann dadurch gebildet sein, daß der Aunddraht 6 einseitig derart zugeschliffen ist, daß von ihm - bei Betrachtung des Querschnitts - Reißabschnitte 9 abgeschliffen sind und die verbleibenden, die Außenwandung bildenden Sehnen (bei Betrachtung des Querschnitts) 11 in einer scharfen Außenkante 8 zulaufen. Die Sehnen 11 können an der Kante 8 einen rechten Winkel bilden, vorzugsweise ist aber ein spitzer Winkel vorgesehen. Insbesondere ein solcher ist dadurch erzielbar, daß der Draht 6 entlang einer Längsseite zu einer im Querschnitt eine Spitze 12 bildenden Kante verpreßt oder verquetscht ist, wie dies der Figur 3 entnehmbar ist. Grundsätzlich kann statt eines Runddrahtes auch ein Flachdraht zu einer Arbeitsdrahtwendel geformt sein, wobei die Schmalseiten des Drahtes zur Achse A der Arbeitswendel hin- bzw. von dieser fortgerichtet sind und insbesondere die äußere Schmalseite ebenfalls wieder zu einer scharfen Kante gebildet sein kann, beispielsweise durch Anschliff oder Verquetschen.

Bei der Figur 4 weist die am distalen Ende 3 der Arbeitswendel 2 befestigte Kugel 4 einen parallel zur Symmetrieachse A der Arbeitswendel, aber exzentrisch zu dieser verlaufende Bohrung 21 auf, durch die sich ein Führungsdraht 22 erstreckt, der ebenfalls durch die einzelnen Windungen der Arbeitswendel exzentrisch zu deren Achse geführt ist. Beim Antrieb der Arbeitswendel 2 über die Antriebswelle 1 zwingt der Führungsdraht 22 die Kugel dazu, daß sie sich nicht um ihre Mittelachse (die mit der Symmetrieachse der Arbeitswendel 2 zusammenfällt), sondern vielmehr exzentrisch um den Führungsdraht 22 dreht. Hierdurch erhält die Arbeitswendel 2 eine Drehachse 23, die in der Figur 4 gestrichelt dargestellt ist, und die im Bereich der Antriebswelle 1 in diese und damit die Symmetrieachse der Wendel 2 übergeht, im Bereich der Kugel aber in den Führungsdraht 22 übergeht und im Zwischenbereich der Arbeitswendel zwischen beiden, also zwischen der Symmetrieachse A und dem Führungsdraht 22 verläuft. Hierdurch wird der Arbeitskanal der Arbeitswendel 2 vergrößert oder verbreitert, womit das Risiko für eine Dissektion oder antegrade Taschenbildung reduziert oder eliminiert wird. Es ergibt sich eine verbesserte und kontrollierte Abrasion der der Wandpartien, insbesondere in Verbindung mit der Ausgestaltung der Figuren 1 bis 3. Der Arbeitsdurchmesser wir gegenüber dem Wendeldurchmesser auf den doppelten Durchmesser des maximalen radialen Abstandes der Wendelaußenseite zum Führungsdraht 22 vergrößert, wobei gleichzeitig das eingeführte Operationsmaterial so kleinlumig wie möglich bleibt, das heißt, eine kleine Punktionsstelle bei großlumigem Arbeitskanal erreichbar ist.

Bei der erfindungsgemäßen Ausgestaltung der Figuren 5 und 6 ist die distale Kugel 4 über einen Teilbereich ihrer zur Achse A der Arbeitswendel 2 parallelen Höhe mit unter einem endlichen Winkel zu ihren von der Achse A ausgehenden Radien mit Einschnitten 31 versehen, wodurch im bezüglich der Drehrichtung B gegebenen rückwärtigen Übergangsbereich zwischen Außenwandung 32 und den Einschnitt begrenzender Wandung 33 eine scharfe Schneidkante 34 gebildet ist. Beim Drehen der Kugel 4 über die Antriebswelle 1 in Richtung des Pfeiles B greift die Kante 34 an den im Gefäß befindlichen Ablagerungen, Plaques etc. an und schält diese ab, wobei durch die ansonsten bis auf die Einschnitte 31 verbleibende kugelförmige Oberfläche der Kugel 4 und den relativ spitzen Winkel der Schneidkante 34 zu der Kugeloberfläche 32 tiefe Einschnitte verhindert werden und der Schneidvorgang lediglich auf ein schonendes Schälen beschränkt bleibt.

Die Figuren 7 und 8 zeigen eine andere Ausgestaltung, bei der die Arbeitswendel 2 durch ein mehrfach um seine Längsachse L schraubenförmig verwendeltes Blatt 41 gebildet ist, an dessen distalem Ende 42 wiederum eine Kugel 4 befestigt ist, während die Arbeitswendel 2 in ihrem proximalen Bereich 43 mit der Antriebswelle 1 verbunden ist. Auch bei einer solchen Arbeitswendel 2 aus einem Blatt 41 können wieder scharfe Außenkanten 8 vorgesehen sein, die aufgrund der Drehrichtung D der Arbeitswendel 2 derart, daß gelöstes Plaques-Material durch diese zur Antriebswelle 1 gefördert wird, sind die Kanten 8 leicht in Arbeitsrichtung gebogen, so daß sie ebenfalls unter einem endlichen Winkel an den abzutragenden Ablagerungen angreifen, ähnlich wie die Kanten 34 der Ausgestaltung der Figuren 5 und 6.

## Patentansprüche

1. Vorrichtung zur Entfernung von Ablagerungen, wie Plaques in Gefäßen, arteriosklerotisch umgewandelte Wandpartien oder dergleichen, mit einer sich am vorderen Ende eines drehbaren Drahtes (1) erstreckenden Arbeitswendel (2), die an ihrem distalen Ende (3) mit einer Kugel (4) versehen ist, dadurch gekennzeichnet, daß die Kugel (4) mit scharfen Kanten (34) versehen ist und daß die scharfen Kanten (34) sich nur über einen Teil der zur Achse (A) der Arbeitswendel (2) parallelen Gesamthöhe der Kugel (4) erstrecken.

2. Vorrichtung nach Anspruch 1, dadurch gekennzeichnet, daß die scharfen Kanten (34) durch die in Drehrichtung (B) der Kugel (4) gesehene rückwärtige Übergangskante zwischen Außenmantel (32) der Kugel (4) und der Begrenzungswandung (33) eines unter einem spitzen Winkel zur Tangente an die Außenwandung (32) in der Kugel (4) gebildeten Schlitzes (31) gebildet ist.

3. Vorrichtung nach Anspruch 1 oder 2, dadurch gekennzeichnet, daß die Außenseite der Arbeitswendel (2) eine scharfe Kante (8) aufweist.

4. Vorrichtung nach Anspruch 3, dadurch gekennzeichnet, daß die scharfe Kante durch Zuschliff des Außenbereichs der Arbeitswendel (2) gebildet ist.

5. Vorrichtung nach Anspruch 3, dadurch gekennzeichnet, daß die scharfe Kante durch Quetschung des Außenbereichs der Arbeitswendel (2) gebildet ist.

6. Vorrichtung nach einem der Ansprüche 1 bis 5, dadurch gekennzeichnet, daß die Arbeitswendel (2) durch einen schraubenförmig geführten Draht gebildet ist.

7. Vorrichtung nach einem der Ansprüche 1 bis 6, dadurch gekennzeichnet, daß die Arbeitswendel (2) durch ein mehrfach um seine Längsachse (L) verdrehtes Blatt (41) gebildet ist.

8. Vorrichtung nach einem der vorangehenden Ansprüche, dadurch gekennzeichnet, daß die Kugel exzentrisch zur Achse des drehbaren Drahts angeordnet ist.

9. Vorrichtung nach einem der Ansprüche 1 bis 8, dadurch gekennzeichnet, daß die Arbeitswendel leicht gekrümmt ist.

10. Vorrichtung nach einem der Ansprüche 1 bis 9, dadurch gekennzeichnet, daß die Kugel exzentrisch zum vorderen Ende der Arbeitswendel angeordnet ist.

11. Vorrichtung nach Anspruch 10, dadurch gekennzeichnet, daß die Kugel (4) parallel zur Symmetrieachse der Arbeitswendel (2), aber exzentrisch zu dieser, eine Bohrung (21) aufweist, durch die sich ein stationär gehaltener Führungsdraht (22) erstreckt.

## Claims

1. Device for removing deposits, such as plaque in vessels, arteriosclerotically transformed wall portions or the like, with a working helix (2) extending at the front end of a rotary wire (1) and which is provided at its distal end (3) with a ball (4), characterized in that the ball (4) is provided with sharp edges (34) and that the sharp edges (34) only extend over part of the total height of the ball (4), which is parallel to the axis (A) of the working helix (2).

2. Device according to claim 1, characterized in that the sharp edges (34) are formed by the rear transition edge between the outer jacket (32) of the ball (4) considered in the rotation direction (B) of said ball (4) and the boundary wall (33) of a slot (31) formed in the ball (4) under an acute angle to the tangent on the outer wall (32).

3. Device according to claim 1 or 2, characterized in that the outside of the working helix (2) has a sharp edge (8).

4. Device according to claim 3, characterized in that the sharp edge is formed by grinding the outer area of the working helix (2) so as to form an edge.

5. Device according to claim 3, characterized in that the sharp edge is formed by squeezing the outer area of the working helix (2).

6. Device according to one of the claims 1 to 5, characterized in that the working helix (2) is formed by a helically guided wire.

7. Device according to one of the claims 1 to 6, characterized in that the working helix (2) is formed by a sheet (41) multiply turned about its longitudinal axis (L).

8. Device according to any one of the preceding claims, characterized in that the ball is positioned eccentrically to the axis of the rotary wire.

9. Device according to one of the claims 1 to 8, characterized in that the working helix is slightly curved.

10. Device according to one of the claims 1 to 9, characterized in that the ball is positioned eccentrically to the front end of the working helix.

11. Device according to claim 10, characterized in that the ball (4) parallel to the axis of symmetry of the working helix (2), but eccentrically thereto, has a hole (21) through which extends a guide wire (22) held in stationary manner.

## Revendications

1. Dispositif pour l'enlèvement de dépôts, tels que des plaques, formés dans les vaisseaux, de portions de parois atteintes par l'artériosclérose ou similaires, comprenant une spirale de travail (2) s'étendant à l'extrémité proximale d'un fil métallique (1) rotatif, munie à son extrémité distale (3) d'une sphère (4), caractérisé en ce que la sphère (4) comporte des arêtes vives (34) et en ce que celles-ci ne s'étendent que sur une partie de la hauteur totale de la sphère (4), parallèlement à l'axe (A) de la spirale de travail (2).

2. Dispositif selon la revendication 1, caractérisé en ce que les arêtes vives (34) sont formées par l'arête de jonction dirigée vers l'arrière par rapport au sens de rotation (B) de la sphère (4) entre l'enveloppe (32) de la sphère (4) et la paroi de délimitation (33) d'une fente (31) formée dans la sphère (4) selon un angle aigu par rapport à la tangente à la paroi externe (32).

3. Dispositif selon l'une quelconque des revendications 1 ou 2, caractérisé en ce que la face extérieure de la spirale de travail (2) présente une arête vive (8).

4. Dispositif selon la revendication 3, caractérisé en ce que l'arête vive est formée par affûtage de la zone externe de la spirale de travail (2).

5. Dispositif selon la revendication 3, caractérisé en ce que l'arête vive est formée par sertissage de la zone externe de la spirale de travail (2).

6. Dispositif selon l'une quelconque des revendications 1 à 5, caractérisé en ce que la spirale de travail (2) est un fil métallique conformé en hélice.

7. Dispositif selon l'une quelconque des revendications 1 à 6, caractérisé en ce que la spirale de travail (2) est formée d'une feuille (4) enroulée plusieurs fois sur elle-même autour de son axe longitudinal (L).

8. Dispositif selon l'une quelconque des revendications précédentes, caractérisé en ce que la sphère est montée excentrée par rapport à l'axe du fil métallique rotatif.

9. Dispositif selon l'une quelconque des revendications 1 à 8, caractérisé en ce que la spirale est légèrement coudée.

10. Dispositif selon l'une quelconque des revendications 1 à 9, caractérisé en ce que la sphère est montée excentrée par rapport à l'extrémité proximale de la spirale de travail.

11. Dispositif selon la revendication 10, caractérisé en ce que la sphère (4) présente un perçage (21), parallèle à l'axe de symétrie de la spirale de travail (2) mais excentré par rapport à celle-ci, perçage à travers lequel passe un fil métallique de guidage (22) maintenu immobile.
